# EUROPEAN PATENT APPLICATION

(11) **EP 4 410 328 A1**
(43) Date of publication of application: **07.08.2024**
(21) Application number: 22876660.6
(22) Date of filing: 08.08.2022
(51) Int. Cl.: A61M 5/168, G08B 21/18, G08B 7/06, G16H 50/30, G16H 50/20, G16H 10/60

(54) **METHOD FOR DIFFERENTIALLY PROVIDING ALARM, AND RECORDING MEDIUM**

(30) Priority: 30.09.2021 KR 20210129438; 30.09.2021 KR 20210130453
(71) Applicant: Eoflow Co., Ltd., Seongnam-si, Gyeonggi-do 13605 (KR)
(72) Inventor: KIM, Ji Hwan, Seoul 07592 (KR); PAIK, Ji Yun, Seongnam-si Gyeonggi-do 13522 (KR); KIM, Moon Bo, Seongnam-si Gyeonggi-do 13309 (KR); LEE, Dong Min, Seongnam-si Gyeonggi-do 13615 (KR); LEE, In Jae, Seongnam-si Gyeonggi-do 13162 (KR); KANG, Seung Kyu, Seoul 04738 (KR)
(74) Representative: Meissner Bolte Partnerschaft mbB
(86) International application number: PCT/KR2022/011732
(87) International publication number: WO 2023/054885

(57) **Abstract**

The present disclosure relates to an alarm method and a recording medium for differentially generating an alarm, and according to an embodiment, a method, performed by an alarm system linked to a drug injector, of differentially generating an alarm may include: receiving state information from the drug injector; determining priority of an alarm and a type of the alarm based on the received state information of the drug injector and a response scenario corresponding to the state information; and differentially generating an alarm that stimulates at least one of senses of sight, hearing, and touch, based on the type and priority of the alarm, and the response scenario.

## Description

### Technical Field

The present disclosure relates to a method and recording medium for differentially providing an alarm.

### Background Art

There may be many techniques for controlling wearable devices such as smart health products or smart watches, and generating related alarms. However, electronic devices, which are related to human safety or risk and may be applied for medical purposes, may require more stringent procedures, certification, etc. for regulation.

For example, the international standard IEC 60601 published by the International Electrotechnical Commission (IEC) on electrical safety of medical devices describes a series of technical standards for the safety and essential performance of medical electrical equipment. Parts 1 to 8 of IEC 60601 specify general requirements for basic safety and essential performance, and general requirements, tests and guidance for alarm systems in medical electrical equipment and medical electrical systems. According to the standard, in order to comply with the IEC standards, which are international standards, medical electrical devices needs to simultaneously provide visual signals and additional alarm signals through auditory, vocal, and tactile means, in providing an alarm system.

In addition, recently, many needs have arisen for designing user-friendly user interfaces (Uls). Design of a user-centered interface refers to creating a user-centered design that matches what a user wants and needs, according to the user's limited abilities and circumstances.

According to this, graphic-based work with design elements is essential for smart health or medical electronic devices to measure biosignals from users and providing data processing results to the users.

### Disclosure

### Technical Problem

The present disclosure provides a method and recording medium for differentially providing an alarm. In addition, the present disclosure provides a method and recording medium for providing a graphic linked to a blood glucose notification level.

Technical objectives of the present disclosure are not limited to the foregoing, and other unmentioned objectives or advantages of the present disclosure would be understood from the following description and be more clearly understood from the embodiments of the present disclosure. In addition, it would be appreciated that the objectives and advantages of the present disclosure may be implemented by means provided in the claims and a combination thereof.

### Technical Solution

A first aspect of the present disclosure provides a method, performed by an alarm system linked to a drug injector, of differentially generating an alarm, the method including: receiving state information from the drug injector; determining priority of an alarm and a type of the alarm based on the received state information of the drug injector and a response scenario corresponding to the state information; and differentially generating an alarm that stimulates at least one of senses of sight, hearing, and touch, based on the priority of the alarm, the type of the alarm, and the response scenario.

A second aspect of the present disclosure provides a method of providing a graphic linked to a blood glucose notification level, the method including: obtaining current blood glucose data of a user through a blood glucose sensor; calculating an expected blood glucose value over time based on the current blood glucose data of the user and an insulin duration for the user; classifying a blood glucose notification level according to the expected blood glucose value at a particular time point at which a predetermined time period elapses from a current time point; and outputting a graphic linked to the blood glucose notification level through a display screen.

### Advantageous Effects

According to an embodiment of the present disclosure, an alarm system of the present disclosure may comply with the international standards International Electrotechnical Commission (IEC) 60601-1-8 and 2-24 and the Korean standard KS C IEC 60601-1-8 and 2-24 based thereon.

The present disclosure implements an intelligent alarm system for determining priority of an alarm and a type of the alarm according to a response scenario, and applying different alarm expression methods according to the response scenario.

According to an embodiment of the present disclosure, the alarm expression method may be changed based on the type and priority of the alarm, as well as the response scenario, rather than simply applying a predetermined alarm expression method.

According to an embodiment of the present disclosure, an optimal alarm system may be designed considering an environment in which an alarm of the present disclosure is applied, for example, partially delaying generation of an auditory alarm or canceling an alarm depending on confirmation by a user.

According to another embodiment of the present disclosure, the present disclosure may provide a user-friendly user interface.

In addition, according to the present disclosure, a processor may provide a graphic to be provided to a user while dynamically modifying the graphic according to an expected increase or decrease in a blood glucose value of the user.

For example, according to the present disclosure, a graphic representing an emoticon accompanied by a change in a facial expression according to a blood glucose state may be provided.

In addition, according to the present disclosure, a user may easily recognize, in addition to a graphic that simply create an aesthetic effect, a risk of blood glucose that may occur after a while, based on blood glucose data measured from the user and an expected blood glucose value after 30 minutes.

### Description of Drawings

FIG. 1 is a flowchart of a method of differentially providing an alarm, according to an embodiment of the present disclosure.
FIGS. 2A and 2B are examples of setting user settings for an alarm, according to an embodiment of the present disclosure.
FIG. 3 is an example for describing the content of an alarm provided by an alarm system, according to an embodiment of the present disclosure.
FIG. 4 is an example for describing priority of an alarm, a type of the alarm, and an alarm expression method, according to an embodiment.
FIG. 5 is an example of an alarm system determining priority of an alarm and a type of the alarm, and selecting an alarm expression method, according to an embodiment.
FIG. 6 is another example of an alarm system determining priority of an alarm and a type of the alarm, and selecting an alarm expression method, according to another embodiment.
FIG. 7 is a flowchart of a method of providing a graphic linked to a blood glucose notification level, according to an embodiment of the present disclosure.
FIG. 8 is an example for describing blood glucose notification levels according to an embodiment of the present disclosure.
FIG. 9 is an example of providing graphics linked to blood glucose notification levels, according to an embodiment of the present disclosure.
FIG. 10 is an example for describing providing blood glucose notification cards according to the passage of a predetermined time period, according to an embodiment of the present disclosure.
FIG. 11 is another example of providing graphics linked to blood glucose notification levels, according to another embodiment of the present disclosure.
FIG. 12 is an example of providing graphics linked to boundaries of blood glucose notification levels, according to another embodiment of the present disclosure.
FIGS. 13A and 13B are examples of augmented reality (AR) emojis according to another embodiment of the present disclosure.

### Best Mode

A first aspect of the present disclosure provides a method, performed by an alarm system linked to a drug injector, of differentially generating an alarm, the method including: receiving state information from the drug injector; determining priority of an alarm and a type of the alarm based on the received state information of the drug injector and a response scenario corresponding to the state information; and differentially generating an alarm that stimulates at least one of senses of sight, hearing, and touch, based on the priority of the alarm, the type of the alarm, and the response scenario.

Here, the receiving of the state information may include receiving state information regarding an operation or an error of the drug injector, through a communication module of the drug injector, or a communication module of a controller linked to the drug injector.

Here, a type of the response scenario may include at least one of a response scenario related to a significant risk to a user's safety, a response scenario requiring an urgent process, a response scenario related to a predicted future situation, and a response scenario for managing the drug injector or the controller.

In addition, the differentially generating of the alarm may include first providing a visual alarm and a tactile alarm according to the determined priority and type of the alarm and then differentially providing an auditory alarm and a visual alarm, or providing an auditory alarm along with the visual alarm and the tactile alarm.

In addition, the differentially generating of the alarm may include: mapping an alarm expression method according to the determined priority and type of the alarm; selectively combining alarm expression methods based on the response scenario; and differentially providing the alarm to a user based on the combined alarm expression methods.

In addition, the method may further include recommending an alarm providing method setting suitable for a user based on the user's alarm checking habits.

A second aspect of the present disclosure provides a method of providing a graphic linked to a blood glucose notification level, the method including: obtaining current blood glucose data of a user through a blood glucose sensor; calculating an expected blood glucose value over time based on the current blood glucose data of the user and an insulin duration for the user; classifying a blood glucose notification level according to the expected blood glucose value at a particular time point at which a predetermined time period elapses from a current time point; and outputting a graphic linked to the blood glucose notification level through a display screen.

Here, the blood glucose sensor may be a continuous blood glucose meter to be attached to a human body to continuously measure blood glucose in the human body.

In addition, the calculating of the blood glucose value may include predicting an insulin duration for the user based on a plurality of pieces of blood glucose data obtained through the blood glucose sensor and user information that is input from the user, and calculate the blood glucose value by using a result of the predicting.

In addition, the classifying of the blood glucose notification level may further include presetting a threshold range of blood glucose values corresponding to a hypoglycemic state, a normal state, or a hyperglycemic state, and setting a blood glucose notification level that is mapped to a notification section, a notification method, and notification intensity for indicating a level of risk according to the state.

In addition, the graphic may be a visual indicator that changes in association with the blood glucose notification level as the expected blood glucose value changes over time.

In addition, the graphic may be an emoticon accompanied by a change in a facial expression, and the outputting of the graphic linked to the blood glucose notification level may include expressing a change in the facial expression of the emoticon in association with the blood glucose notification level.

In addition, the graphic may be an indicator having a shape of an arrow, and the outputting of the graphic linked to the blood glucose notification level may include determining a size, a color, and a shape of the arrow according to an increase or a decrease in the blood glucose value of the user, and expressing an animation effect of the arrow being added in association with the blood glucose notification level.

In addition, the graphic may be at least one of an avatar, a virtual character, and an augmented reality emoji of the user that are generated by reflecting at least one of an appearance, a shape, features, and preferences of the user, and the outputting of the graphic linked to the blood glucose notification level may include expressing a change in at least one of a facial expression, an action, and a background of the at least one of the avatar, the virtual character, and the augmented reality emoji of the user in association with the blood glucose notification level.

In addition, other methods and systems for implementing the present disclosure, and a computer-readable recording medium having recorded thereon a computer program for executing the methods may be further provided.

### Mode for Invention

Advantages and features of the present disclosure and a method for achieving them will be apparent with reference to embodiments of the present disclosure described below together with the accompanying drawings. The present disclosure may, however, be embodied in many different forms and should not be construed as being limited to the embodiments set forth herein, and all changes, equivalents, and substitutes that do not depart from the spirit and technical scope of the present disclosure are encompassed in the present disclosure. These embodiments are provided such that the present disclosure will be thorough and complete, and will fully convey the concept of the present disclosure to those of skill in the art. In describing the present disclosure, detailed explanations of the related art are omitted when it is deemed that they may unnecessarily obscure the gist of the present disclosure.

Terms used herein are for describing particular embodiments and are not intended to limit the scope of the present disclosure. Singular forms are intended to include plural forms as well, unless the context clearly indicates otherwise. As used herein, terms such as "comprises," "includes," or "has" specify the presence of stated features, numbers, stages, operations, components, parts, or a combination thereof, but do not preclude the presence or addition of one or more other features, numbers, stages, operations, components, parts, or a combination thereof.

Some embodiments of the present disclosure may be represented by functional block components and various processing operations. Some or all of the functional blocks may be implemented by any number of hardware and/or software elements that perform particular functions. For example, the functional blocks of the present disclosure may be embodied by at least one microprocessor or by circuit components for a certain function. In addition, for example, the functional blocks of the present disclosure may be implemented by using various programming or scripting languages. The functional blocks may be implemented by using various algorithms executable by one or more processors. Furthermore, the present disclosure may employ known technologies for electronic settings, signal processing, and/or data processing. Terms such as "mechanism", "element", "unit", or "component" are used in a broad sense and are not limited to mechanical or physical components.

In addition, connection lines or connection members between components illustrated in the drawings are merely exemplary of functional connections and/or physical or circuit connections. Various alternative or additional functional connections, physical connections, or circuit connections between components may be present in a practical device.

Hereinafter, the present disclosure will be described in detail with reference to the accompanying drawings.

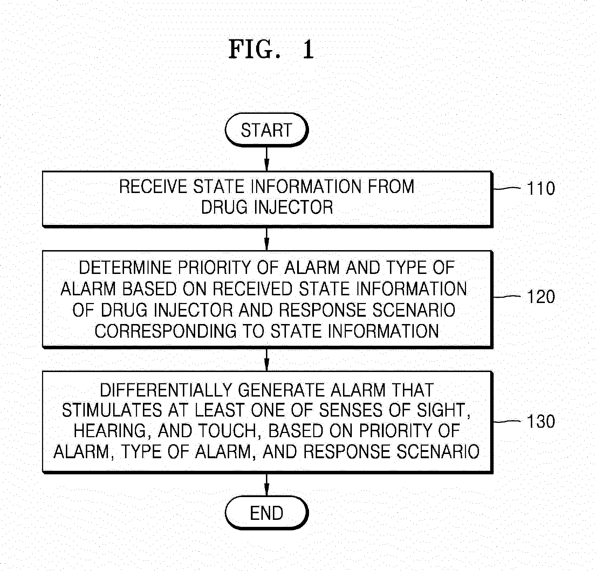

FIG. 1 is a flowchart of a method of differentially providing an alarm, according to an embodiment of the present disclosure.

An alarm system according to an embodiment of the present disclosure may be performed by a user terminal including a processor configured to perform calculations, such as a computer, a mobile device, or a server. Here, the user terminal may refer to a communication terminal that may use a web service in a wired or wireless communication environment. For example, the user terminal may be a user's personal computer or portable terminal. In the drawings, the portable terminal is illustrated as a smart phone, but the spirit of the present disclosure is not limited thereto, and as described above, any terminal equipped with an application for performing short-range wireless communication and Internet communication may be used without restrictions.

As another example, the alarm system of the present disclosure may communicate with a drug injector or a controller of the drug injector by using a short-range wireless communication method. For example, the alarm system may receive state information from the drug injector by using Bluetooth or the like, or may receive information about an operation and control of the drug injector from the controller of the drug injector.

In operation 110, the alarm system may receive state information from the drug injector. Here, the drug injector is a device that is to be attached to a patient's body to inject a drug or that includes a sensor to sense the patient's condition, in order to treat a particular disease, and may be a patch-type drug infusion pump.

However, the present disclosure is not limited to a drug injected by the drug injector or the type of the device, and hereinafter, the drug injector may be described as a terminal that provides device state information and processes sensitive information about a user, which is necessary to implement the present disclosure.

The drug injector may require continuous maintenance to treat a disease or to manage the user's health. For example, the controller or the user terminal may manage the drug injector to operate in a normal state by scheduling an operation of the drug injector and monitoring the state of the drug injector. To this end, the drug injector may transmit state information to the user terminal via the controller or a web application through a communication module. Meanwhile, hereinafter, a patch-type insulin pump for managing blood glucose of diabetic patients will be described as an example of the drug injector.

In the user terminal, the alarm system may receive state information through a communication module of the drug injector or a communication module of the controller linked to the drug injector. Here, the state information may include information about an operation or an error of the drug injector.

Here, the alarm system may receive state information regarding the operation or error of the drug injector through the communication module of the drug injector or the communication module of the controller linked to the drug injector.

For example, the alarm system may receive state information regarding a drug administration scheduling operation of the drug injector. For example, the alarm system may receive, from the drug injector or the controller, state information about an scheduling operation, such as a time point of beginning of drug injection by the drug injector, the amount of drug to be administered, a time point of completion of the drug injection, and a time point of next drug injection.

As another example, the alarm system may receive state information regarding an error of the drug injector or the controller. For example, the alarm system may receive, from the drug injector or the controller, state information such as a failure of the drug injector, interruption of drug injection, absence of a drug, disposal of the drug injector, blockage of an injection hole, improper temperature, needle insertion error, absence of a battery of the drug injector, or a control app error.

In addition, the alarm system may receive, from the drug injector, state information such as exceeding an injection stop time, interruption of automatic drug injection, imminent expiration of the service life of the drug injector, or a low state of charge of a battery of the drug injector.

Meanwhile, the alarm system may receive state information such as absence of a battery of the controller, overheating of the controller, or a low state of charge of a battery of the controller.

In operation 120, the alarm system may determine priority of an alarm and a type of the alarm based on the received state information of the drug injector and a response scenario corresponding to the state information. To this end, the alarm system may control the drug injector in response to the state information of the drug injector, and set in advance a plurality of response scenarios regarding alarms to be provided to the user. For example, when the alarm system receives state information from the drug injector or the controller, the alarm system may match a response scenario to the state information and determine priority of an alarm and a type of the alarm.

For example, the alarm system may determine the priority based on the content of the response scenario. For example, types of response scenarios may include at least one of response scenarios related to a significant risk to the user's safety, response scenarios requiring an urgent process, response scenarios related to predicted future situations, and response scenarios for managing the drug injector or the controller.

Here, the alarm system may determine the priority for the alarm based on the response scenario that matches the received state information. For example, when a scheduled drug is not injected through the drug injector, is not injected in an insufficient amount, or is injected in an excessive amount, it may cause a dangerous situation for the user and thus pose a significant risk to the user's safety, and the alarm system may determine the highest priority for a response scenario associated with a significant risk to the user's safety.

As another example, there may be a case where an operation of the drug injector needs to be quickly controlled in order to administer a certain amount of drug to the user. For example, when the alarm system receives, from the drug injector, state information such as interruption of automatic drug injection, insufficient amount of drug, self-diagnosis of injection hole blockage, or needle insertion error, the alarm system may match the state information to a response scenario for a matter requiring an urgent process, and selectively determine whether the response scenario is a high or medium priority.

As another example, the alarm system receives, from the drug injector or the controller, state information about an error of the drug injector such as a drug injector authentication error, a controller authentication error, a low state of charge of a battery, absence of a battery, expiration of the service life of the drug injector, disposal of the drug injector, or overheating, the alarm system may match the state information to a response scenario for a matter predicted to occur in the future or a response scenario for a matter for managing the drug injector and/or the controller, and selectively determine whether the response scenario is an intermediate priority or no priority.

In addition, the alarm system may classify types of alarms into Alarm, Alert, and Reminder. Here, the type of an alarm may be type information and may be determined according to a response stage of the alarm. When a new response scenario corresponding to state information of the drug injector is added, the alarm system may add, modify, or delete a type of alarm.

In operation 130, the alarm system may differentially generate an alarm that stimulates at least one of senses of sight, hearing, and touch, based on the priority of the alarm, the type of the alarm, and the response scenario. For example, according to a predetermined alarm generation cycle, the alarm system may differentially generate, an alarm that stimulates a sense of sight, an alarm that stimulates a sense of hearing, an alarm that stimulates a sense of touch, an alarm that stimulates senses of sight and hearing, an alarm that stimulates senses of hearing and touch, an alarm that stimulates senses of hearing and touch, and an alarm that stimulates senses of sight, hearing, and touch, and the like.

A user terminal may include its own alarm providing method (e.g., sound, vibration, or silence), but when using a medical electronic device as in the present disclosure, it may be necessary to determine the priority of an alarm independently of the alarm providing method of the user terminal, or to generate an alarm regardless of an alarm providing method in the system.

In addition, in general, in order for medical electronic devices to comply with the Korean standard KS, the international standard IEC 60601-1-8, or the like, alarm providing methods including a method of stimulating all of senses of sight, hearing, and touch may be recommended. However, when an alarm is provided in a way that stimulates all of senses of sight, hearing, and touch, the alarm may be provided against the user's will, causing inconvenience to the user.

In contrast, according to the alarm system of the present disclosure, an alarm may be provided confidentially to the user in association with a drug injector. According to an embodiment of the present disclosure, the alarm system may first provide a visual alarm and a tactile alarm through a display screen of the user terminal and then differentially provide an auditory alarm, based on determined priority.

For example, the alarm system may map alarm expression methods to determined priorities of alarms and types of the alarms, selectively combine the alarm expression methods based on a response scenario, and differentially provide an alarm to the user based on the combined alarm expression methods.

For example, when generating a visual alarm, the alarm system may differently configure an alarm providing method, for example, a light (e.g., a light-emitting diode (LED)) to be turned on may be expressed differently in color according to the response scenario, such as RED, YELLOW, or BLUE, or the light may be simply turned on or flickers.

As another example, when generating an auditory alarm, the alarm system may differently set a generation time point and duration of a sound, an order of the sound with respect to other alarms, and the like, depending on the response scenario. For example, the alarm system may set an alarm, including a start time, a pause time, and a restart time of generation of a sound, for outputting the sound for 4 seconds and then pausing for 4 seconds. Here, the alarm system may determine a sound reproduction method based on the type and priority of the alarm, and the response scenario.

As another example, an alarm system may generate a vibration as a tactile alarm. Here, the alarm system may set the number of vibrations, and a vibration method, such as vibration intensity, a vibration time, a pause time, and a re-vibration time, differently depending on the response scenario.

Meanwhile, the alarm system may combine or select various expression methods for the above methods of providing visual, auditory, and tactile alarms, and thus provide the user with alarms by using different expression methods depending on a response scenario, even when the alarms belong to the same type and have the same priority.

For example, according to a determined type and priority of an alarm, the alarm system may first provide a visual alarm and a tactile alarm, and then differentially provide an auditory alarm and a visual alarm or provide an auditory alarm along with a visual alarm and a tactile alarm.

According to this, the method, performed by which the alarm system of the present disclosure, of generating an alarm is more diverse than existing methods of providing an alarm. In addition, the alarm system of the present disclosure prioritizes an alarm for a more serious and urgent matter and thus set the importance of an alarm to be provided to the user differently depending on a response scenario, thereby providing the user with a more effective alarm.

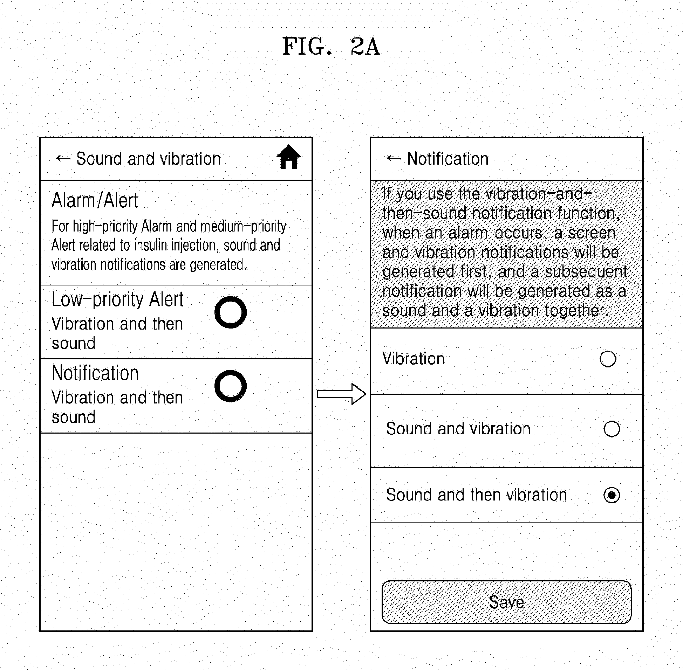

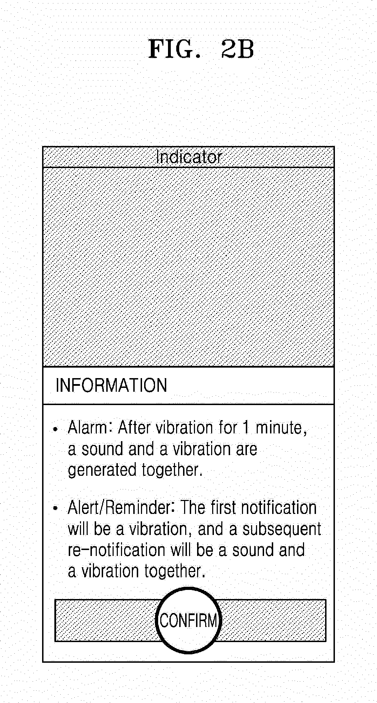

FIGS. 2A and 2B are examples of setting user settings for an alarm, according to an embodiment of the present disclosure. According to an embodiment of the present disclosure, the alarm system may be provided to the user in a visual manner through a display screen of the user terminal.

By setting an alarm expression method, the user may specify in advance the alarm expression method to be provided from the alarm system.

Referring to FIG. 2A, in a "Sound and vibration" window, the alarm system may inform the user in advance that alarms of the types 'Alarm' and 'Alert' are always provided through a sound and a vibration. Alternatively, when the user selects a "Notification" window, the user may select "Sound", "Sound and vibration", "Vibration and then sound", or the like, as a method of providing an alarm, in the "Notification" window.

Meanwhile, when there is no separate alarm expression method setting from the user, the alarm system may set "Vibration and then sound" as a default setting.

In addition, the alarm system of the present disclosure may further include an operation of recommending an alarm providing method setting suitable for the user based on the user's alarm checking habits.

Meanwhile, referring to FIG. 2B, guidance on the "Vibration and then sound" item may be provided. For example, the alarm system may provide information such as "Alarm: After a vibration for 1 minute, a sound and a vibration will be generated together". As another example, the alarm system may provide information such as "Alarm/Reminder: A vibration will be generated as the first notification, and a sound and a vibration will be generated as a subsequent re-notification".

However, this is only an example and does not describe all alarm expression methods that may be selected by an embodiment of the present disclosure. In addition, the types of alarms are arbitrarily classified for describing an embodiment of the present disclosure, and the meaning of the term and the method of expressing an alarm according to the type of an alarm may also be set differently from the above.

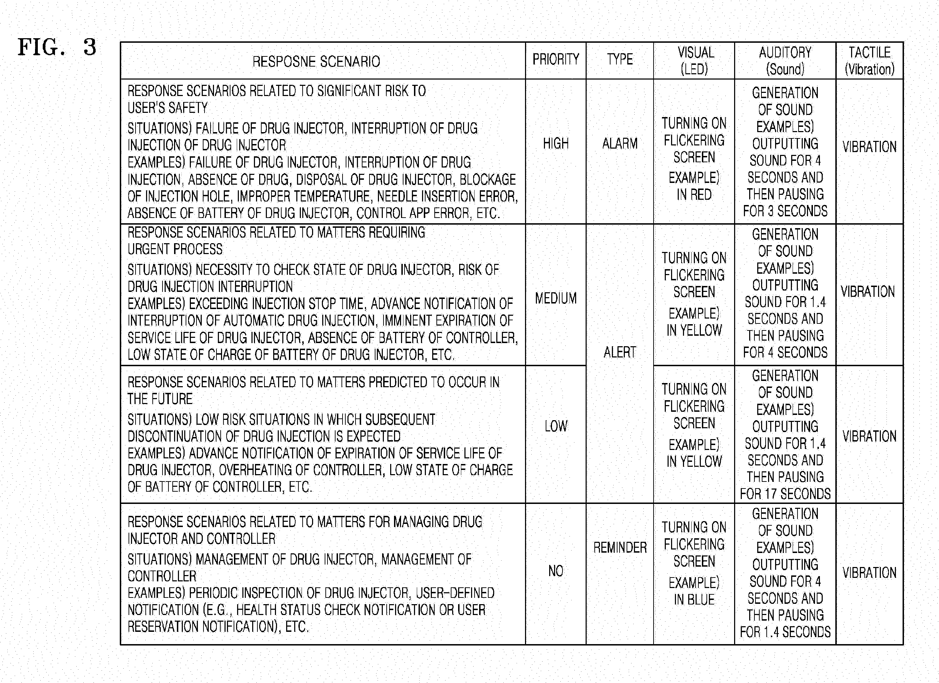

FIG. 3 is an example for describing the content of an alarm provided by an alarm system, according to an embodiment of the present disclosure. However, FIG. 3 is merely an example, and it is also possible to implement the alarm providing method of the present disclosure in a different manner.

According to an embodiment of the present disclosure, the alarm system may determine priority of an alarm and a type of the alarm based on received state information of the drug injector and a response scenario corresponding to the state information. According to an embodiment, the response scenario may be a response scenario for an alarm to be provided to the user in response to the state information received from the drug injector or the controller.

Types of response scenarios may include response scenarios related to a significant risk to the user's safety, response scenarios related to matters requiring an urgent process, response scenarios related to matters predicted to occur in the future, response scenarios related to matters for managing the drug injector and/or the controller, and the like. Here, the types of response scenarios are examples and are created for describing the present disclosure.

For example, the response scenarios related to a significant risk to the user's safety may refer to situations in which the drug injector fails or the drug injector stops injecting a drug. For example, they may include a drug injector failure, interruption of drug injection, absence of a drug, disposal of the drug injector, blockage of an injection hole, improper temperature, a needle insertion error, absence of a battery of the drug injector, a control app error, and the like.

As another example, the response scenarios related to matters requiring an urgent process may refer to situations in which the state of the drug injector needs to be checked or there is a risk of interruption of drug injection. For example, they may include exceeding an injection stop time, advance notification of interruption of automatic drug injection, imminent expiration of the service life of the drug injector, absence of a battery of the controller, a low state of charge of a battery of the drug injector, and the like.

As another example, the response scenarios related to matters predicted to occur in the future may refer to low-risk situations in which a subsequent discontinuation of drug injection is expected. For example, they may include an advance notification of expiration of the service life of the drug injector, overheating of the controller, a low state of charge of a battery of the controller, and the like.

As another example, the response scenarios related to matters for managing the drug injector and the controller may refer to management situations for the drug injector. For example, they may include periodic inspection of the drug injector, a user-defined notification (e.g., a health status check notification or a user reservation notification), and the like.

The alarm system of the present disclosure may determine priority of an alarm and a type of the alarm based on state information of the drug injector and a response scenario corresponding to the state information. For example, response scenarios may be categorized according to the priority of the alarm, and here, the priority of the alarm may be classified into High, Medium, Low, No, and the like. The priority may be classified by using other names than in the embodiments described herein, and may be expressed as a grade or a score.

In addition, the alarm system of the present disclosure may classify types of alarms into Alarm, Alert, and Reminder.

Meanwhile, the alarm system may select an alarm expression method by selecting or combining at least one of visual, auditory, and tactile methods based on the type and priority of the alarm, and the response scenario.

According to embodiments of the present disclosure, it is possible to combine various types of alarm expression methods. For facilitating understanding, in an embodiment, the alarm system of the present disclosure may provide an alarm to the user through turning on a flickering screen as a visual method, generating a sound as an auditory method, and a vibration as a tactile method.

For example, when the priority of the alarm is high and the type of the alarm is Alarm, the alarm system may provide an alarm to the user by turning on a flickering screen in red or the like, or generating a sound for 4 seconds and then pausing for 3 seconds, or generating a vibration, according to the response scenario.

As another example, when the priority of the alarm is medium and the type of the alarm is Alert, the alarm system may provide an alarm to the user by turning on a flickering screen in yellow or the like, or generating a sound for 1.4 seconds and then pausing for 4 seconds, or generating a vibration, according to the response scenario.

As another example, when the priority of the alarm is low and the type of the alarm is Alert, the alarm system may provide an alarm to the user by turning on a screen in yellow or the like, or generating a sound for 1.4 seconds and then pausing for 17 seconds, or generating a vibration, according to the response scenario.

As another example, when the alarm has no priority and the type of the alarm is Reminder, the alarm system may provide an alarm to the user by turning on a screen in blue or the like, or generating a sound for 4 seconds and then pausing for 14.4 seconds, or generating a vibration, according to the response scenario.

In addition, according to an embodiment of the present disclosure, the alarm system may selectively provide a visual alarm, an auditory alarm, and a tactile alarm simultaneously or at different time points. For example, the alarm system may diversify the alarm expression method by simultaneously providing a visual alarm and a tactile alarm and providing an auditory alarm with a predetermined time delay.

According to this, the alarm system may select different alarm providing methods depending on the priority of the alarm, the type of the alarm, and the response scenario, or may provide an alarm to the user by selectively combining several methods.

Meanwhile, the alarm system may configure or combine alarms in a different manner from the embodiments described above. It should be understood that the type and priority of the alarm described above with reference to FIG. 3 are merely an embodiment, and that various modifications of the embodiment within the same scope may also be possible.

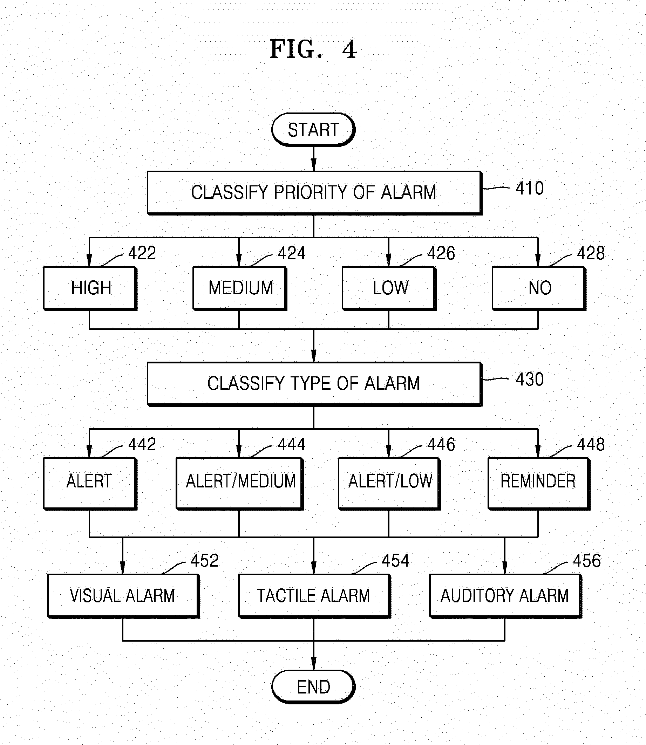

FIG. 4 is an example for describing priority of an alarm, a type of the alarm, and an alarm expression method, according to an embodiment. Referring to FIG. 4, the alarm system may classify priority of an alarm (410) to determine the alarm as High 422, Medium 424, Low 426, or No 428.

In addition, the alarm system may classify the type of the alarm (430) to determine the alarm as Alarm 442, Alert/Medium 444, Alert/Low 446, or Reminder 424.

In addition, the alarm system may generate an alarm by selecting or combining at least one of a visual alarm 452, a tactile alarm 454, and an auditory alarm 456, according to the priority of the alarm and the type of the alarm.

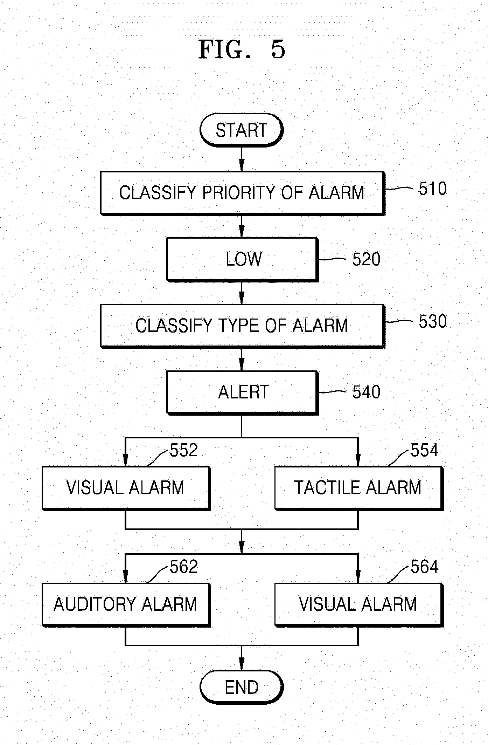

FIG. 5 is an example of an alarm system determining priority of an alarm and a type of the alarm, and selecting an alarm expression method, according to an embodiment. Referring to FIG. 5, the alarm system may classify priority of an alarm (510) to determine the priority of the alarm as Low 520. In addition, the alarm system may classify the type of the alarm (530) to determine the type of the alarm as Alert 540.

In addition, the alarm system may simultaneously provide the user with a visual alarm 552 and a tactile alarm 554, then pause for a time interval, and then simultaneously provide an auditory alarm 562 and a visual alarm 564, as alarm expression methods.

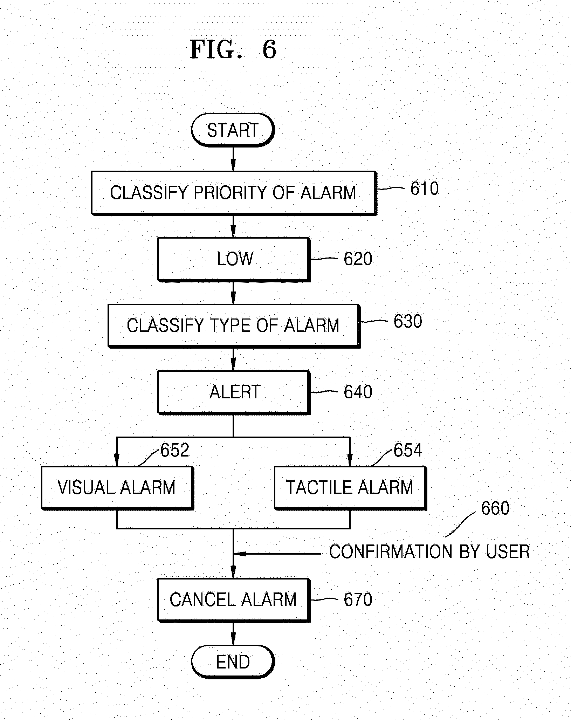

FIG. 6 is an example of an alarm system determining priority of an alarm and a type of the alarm, and selecting an alarm expression method, according to another embodiment. Referring to FIG. 6, the alarm system may classify priority of an alarm (610) to determine the priority of the alarm as Low 620. In addition, the alarm system may classify the type of the alarm (630) to determine the type of the alarm as Alert 620.

In addition, the alarm system may simultaneously provide the user with a visual alarm 652 and a tactile alarm 654, and when the user confirms the alarms (660), cancel an alarm to be provided differentially in the future according to the response scenario (670).

According to embodiments of the present disclosure, the present disclosure may include an alarm regarding sensitive information, and thus prevent undesired exposure of medical information of a user in a public place. In addition, the alarm system of the present disclosure may improve user convenience by determining priority of alarms, and when the user confirms an alarm that is provided first, canceling low-priority alarms that are relatively minor, do not require an urgent response, and are to be differentially provided.

Hereinafter, the present disclosure will be described in detail with reference to the accompanying drawings.

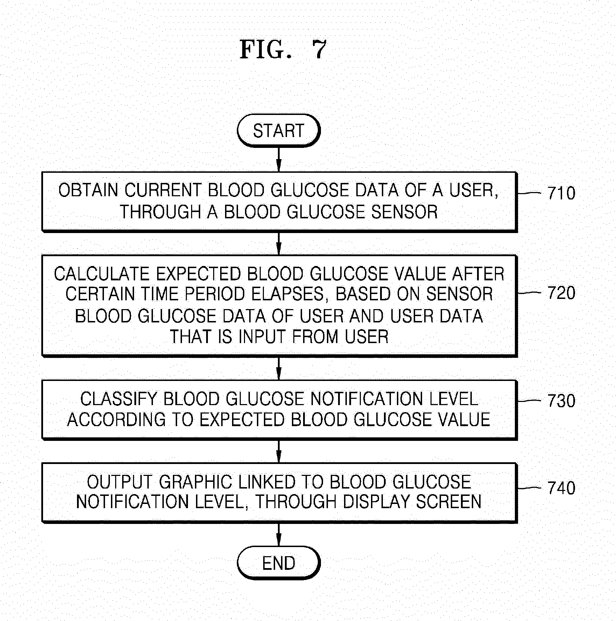

FIG. 7 is a flowchart of a method of providing a graphic linked to a blood glucose notification level, according to an embodiment of the present disclosure.

According to an embodiment of the present disclosure, the method of providing a graphic linked to a blood glucose notification level may be performed by a user terminal including a processor configured to performs calculations, such as a computer, a mobile device, or a server.

Here, the user terminal may refer to a communication terminal that may use a web service in a wired or wireless communication environment. For example, the user terminal may be a user's personal computer or portable terminal. As described above, any terminal equipped with an application for performing Internet communication may be used without restrictions.

In operation 710, the processor may obtain current blood glucose data of a user, through a blood glucose sensor. Here, the blood glucose sensor may be a continuous glucose monitor (CGM) to be attached to a human body to continuously measure blood glucose in the body. For example, the blood glucose sensor may be attached under a user's skin to continuously track the user's blood glucose state. A measured blood glucose value of the user obtained through the blood glucose sensor may be transmitted to the user terminal through a communication module. Meanwhile, the blood glucose sensor may be attached to a living body or may be a wearable device. The blood glucose sensor may be, for example, in the form of a patch.

In operation 720, the processor may calculate an expected blood glucose value over time based on the current blood glucose data of the user and an insulin duration for the user.

For example, the processor may predict the insulin duration for the user based on a plurality of pieces of blood glucose data obtained through the blood glucose sensor and user information that is input from the user, and calculate a blood glucose value by using a result of the prediction.

An example of a blood glucose prediction module (BG prediction) is the TypeZero Artificial Pancreas algorithm, which may measure sensor glucose (SG) values at 5-minute intervals through a continuous blood glucose meter, and predict an insulin duration for the user from user information such as the user's height and weight. The TypeZero Artificial Pancreas algorithm may calculate a predicted blood glucose (BG) value of the user after about 30 minutes through the above process. In this process, information about the user's height or weight may be considered, and when the information about the user's height or weight is not input, the blood glucose value cannot be calculated, or an inaccurate blood glucose value may be obtained.

Meanwhile, the processor may calculate a blood glucose value for the user by considering the amount of insulin injected into the user through a drug injector and/or the amount of insulin absorbed from food consumed by the user through a meal, in addition to the current blood glucose data of the user measured by the blood glucose sensor.

For example, the processor may calculate the blood glucose value based on at least one of a basal bolus, a correction bolus, and a meal bolus. For example, the processor may automatically calculate a basal bolus and a correction bolus that are injected through a drug injector, and calculate a blood glucose value of the user after 30 minutes based on a meal bolus that is manually input by the user. Here, the time period of 30 minutes is a time period selected for describing an expected future blood glucose value in the present disclosure, and is an arbitrary time period that may vary depending on an embodiment. Meanwhile, because the present disclosure may obtain continuous blood glucose data from the blood glucose sensor, and the drug injector may also automatically calculate drug injection and correction boluses, the processor may continuously calculate blood glucose values at particular time points by using a particular algorithm.

In operation 730, the processor may classify a blood glucose notification level according to an expected blood glucose value at a particular time point at which a predetermined time period elapses from the present. For example, the processor may set a certain threshold section and, when the calculated blood glucose value falls within the range of the certain threshold section, classify the blood glucose notification level according to the expected blood glucose value. Here, the processor may preset a threshold section of blood glucose values corresponding to a hypoglycemic state, a normal state, or a hyperglycemic state, and set a blood glucose notification level that is mapped to a notification section, a notification method, and notification intensity for indicating the risk associated with the hypoglycemic state, the normal state, and the hyperglycemic state.

For example, the processor may set blood glucose notification levels ranging from level 1 to level 5 based on the expected blood glucose value after 30 minutes, and map a notification method and notification intensity corresponding to each level. For example, the processor may map a graphic to be provided to the user and a graphic display method for each level, and store them in a memory (or a database).

In operation 740, the processor may output a graphic linked to the blood glucose notification level, through a display screen. Here, the graphic may be a visual indicator that changes in association with the blood glucose notification level as the expected blood glucose value changes over time.

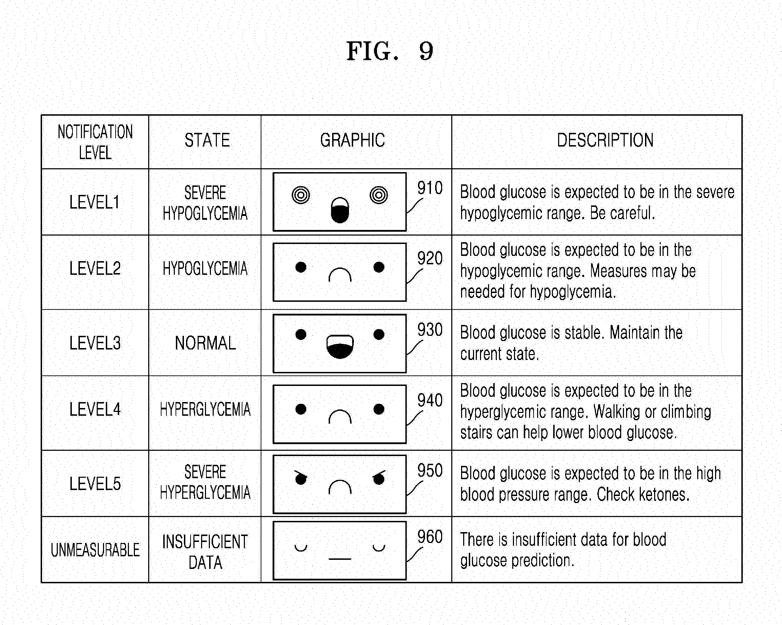

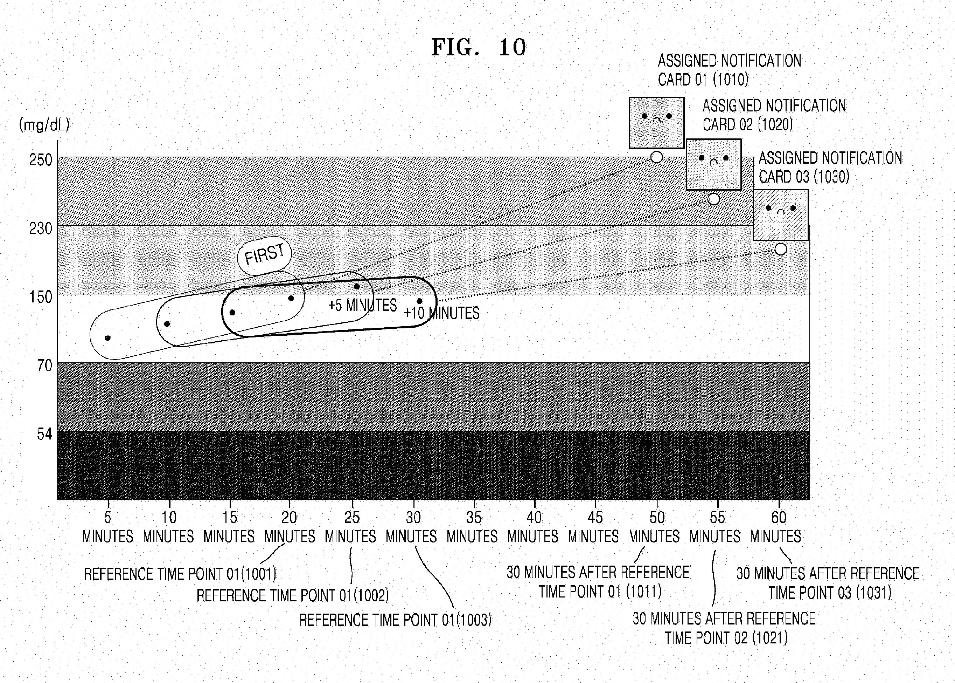

According to an embodiment, the graphic may be a card-type graphic that intuitively represents the blood glucose notification level of the user. When the graphic is referred to as a blood glucose notification card, the processor may provide the user with a newly predicted blood glucose notification card for each preset time unit. For example, the processor may obtain blood glucose data at previous four time points in 5-minute increments and provide the user with a blood glucose notification card for a predicted blood glucose situation after 30 minutes based on the blood glucose data. According to another embodiment, the graphic is an emoticon accompanying a change in a facial expression, and the processor may output a graphic linked to the blood glucose notification level by expressing a change in the facial expression of the emoticon in association with the blood glucose notification level. Embodiments of the above will be described below with reference to FIGS. 9 and 10.

In addition, according to another embodiment, the graphic is an indicator having the shape of an arrow, and the processor may determine the size, color, and shape of the arrow according to an increase or decrease in the blood glucose value of the user, and output the graphic linked to the blood glucose notification level by expressing an animation effect of the arrow added in association with the blood glucose notification level.

For example, the processor may output a graphic corresponding to an upward pointing arrow when the user is expected to be in a hyperglycemic state after 30 minutes, and output a graphic corresponding to a larger upward pointing arrow (than the graphic corresponding to the hyperglycemic state) when the user is expected to be in a severe hyperglycemic state after 30 minutes. Likewise, the processor may output a graphic corresponding to a downward pointing arrow with different sizes in response to a hypoglycemic state or a severe hypoglycemic state.

Meanwhile, by using the above arrows as indicators to simply show whether the blood glucose increases or decreases, or show an increase or decrease in the blood glucose that the user needs to be aware of, rather than using all graphics corresponding to conditions of the user, the processor may allow the user to recognize the current blood glucose state in a visual manner at a glance without having to compare to determine whether the value increases or decreases.

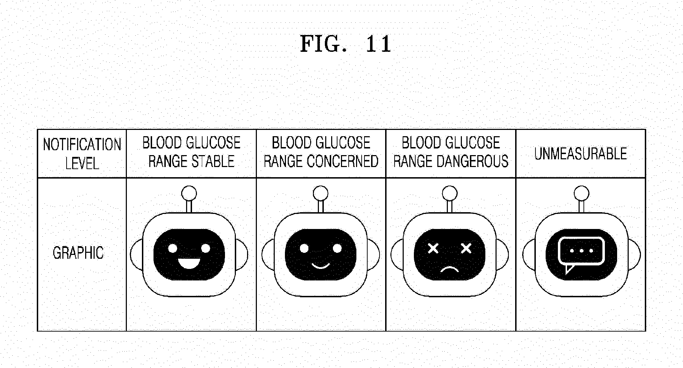

For example, the expected blood glucose value after 30 minutes calculated by the processor may be a dynamic value that changes over time. For example, when there is no separate action of administering insulin to the user, the expected blood glucose value in the user's body may gradually decrease over time. Accordingly, when the blood glucose notification level changes over time, the processor may output a graphic representing a notification of a threshold section. This will be described below with reference to FIG. 11.

By presetting various types of graphics, the processor may provide the user with a blood glucose notification in a more familiar and interesting manner. In addition, the processor may additionally provide a user interface means through which the user may directly generate a graphic to be output.

For example, the graphic may be at least one of the user's avatar, a virtual character, and an augmented reality emoji generated by reflecting at least one of the user's appearance, shape, features, and preferences, and the processor may output a graphic linked to the blood glucose notification level by expressing a change in at least one of the facial expression, action, and background of the at least one of the user's avatar, the virtual character, and the augmented reality emoji in association with the blood glucose notification level. In relation to this, when a picture of the user is input, the processor may generate an avatar corresponding to the input picture, and output a graphic linked to the blood glucose notification level by changing at least one of the avatar's facial expression, action, and background in association with the blood glucose notification level.

Recently, as the number of users expressing themselves through avatars has increased, many techniques that provide methods of providing customized notifications for users have been implemented. According to an embodiment of the present disclosure, the processor may express an expected increase or decrease in the blood glucose value of the user through an action of the user's avatar (e.g., energetic, lethargic, or angry) or a change in the facial expression of the avatar, or an increase or decrease in the blood glucose in situations that may occur related to a blood glucose risk, through an interesting graphic (e.g., hypoglycemia through a collapsing character, or hyperglycemia as an angry character).

As another example, the processor may download a graphic linked to the blood glucose notification level from a server, or obtain the graphic through game content or other applications.

Meanwhile, the graphics described in the present disclosure should only be understood as examples. Aside from copyrights held by content creators, when blood glucose data may be obtained through the blood glucose sensor of the present disclosure and expressed in an appropriate graphic in association with the operation of calculating the expected blood glucose value over time, it may be interpreted as an embodiment having the same and equivalent scope as the present disclosure.

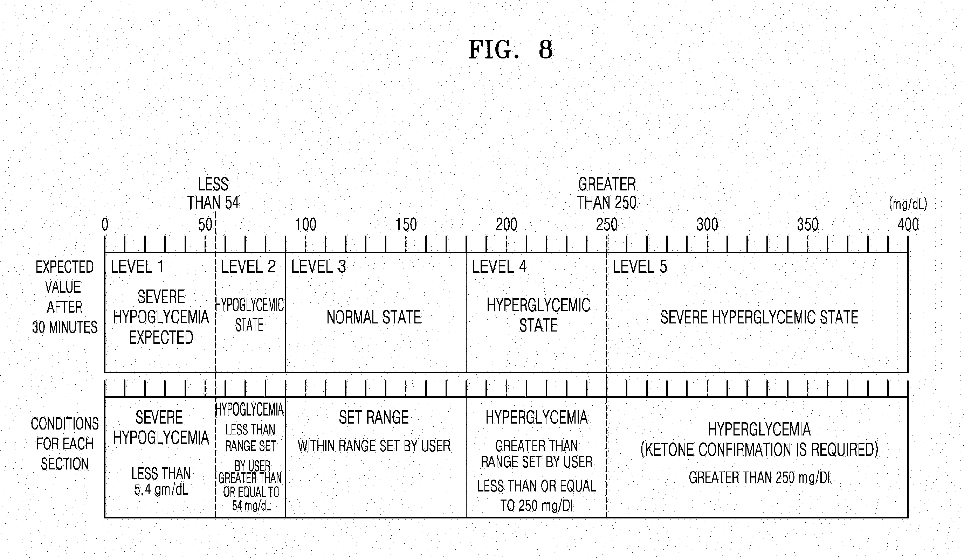

FIG. 8 is an example for describing blood glucose notification levels according to an embodiment of the present disclosure. Referring to FIG. 8, the processor may set five blood glucose notification levels based on an expected blood glucose state of the user after 30 minutes. In addition, the processor may further provide an interface for adjusting a threshold section within a range set by the user, in addition to a threshold section that people generally use to determine a hypoglycemic state or a hyperglycemic state.

First, referring to the upper part of FIG. 8, the processor may specify an expected blood glucose value after 30 minutes, in a section for setting each blood glucose notification level. For example, the processor may specify a level 1 blood glucose notification level for a range of expected blood glucose values of 0 to 54 mg/dL, and expect that the user in this range is in a severe hypoglycemic state. In addition, the processor may specify a level 2 blood glucose notification level for a range of expected blood glucose values of 54 to 90 mg/dL, and expect that the user in this range is in a hypoglycemic state. Similarly, the processor may specify a level 3 blood glucose notification level of expected blood glucose values of 90 to 180 mg/dL, and expect that the user is in a normal state. In addition, the processor may specify a level 4 blood glucose notification level for a range of expected blood glucose values of 180 to 250 mg/dL, and expect that the user is in a hyperglycemic state. In addition, the processor may specify a level 5 blood glucose notification level for a range of expected blood glucose values of 250 to 400 mg/dL, and expect that the user is in a severe hyperglycemic state.

Next, referring to the lower part of FIG. 8, the processor may set conditions for each section. For example, the processor may allow the user to set a range through a user interface and specify a blood glucose notification level based on the set range. However, a severe hypoglycemic state with an expected blood glucose value of less than 54 mg/dL or a severe hyperglycemic state with an expected blood glucose value of greater than 250 mg/dL (ketone confirmation is required) refers to a section that requires a caution or warning notification to the user, and thus, a minimum criterion for the corresponding level may be specified as a section that cannot be changed by the user.

Meanwhile, it is also possible to specify a blood glucose notification level in a manner different from that described with reference to FIG. 8. For example, there may be a plurality of embodiments that may be interpreted as being equivalent to the present disclosure, for example, differently specifying a threshold section for each state, or setting a different number of levels or different level names.

In addition, a blood glucose notification level is classified based on an expected blood glucose value of the user after 30 minutes in the present disclosure, however, when specifying a blood glucose notification level with a different reference time, the threshold sections for an expected blood glucose value for the user and blood glucose notification levels may also vary.

FIG. 9 is an example of providing graphics linked to blood glucose notification levels, according to an embodiment of the present disclosure. Here, the graphic may be expressed as an emoticon accompanied by a change in a facial expression, and an appropriate phrase describing a state of a user may be simultaneously provided to the user along with the graphic.

Meanwhile, FIG. 9 shows emoticons, but in order to further maximize their visual effects, the background color of the graphics corresponding to the respective levels may be changed, or the graphics may be further designed with BG colors, which may indicate emotions of the user.

According to an example, the processor may configure graphics and descriptions corresponding to notification levels from level 1 to level 5 as shown in FIG. 9.

The processor may map, to blood glucose notification level 1, a severe hypoglycemic state, a graphic 910, and a description "Blood glucose is expected to be in the severe hypoglycemic range. Be careful.". In addition, the processor may map, to blood glucose notification level 2, a hypoglycemic state, a graphic 920, and a description "Blood glucose is expected to be in the hypoglycemic range. Measures may be needed for hypoglycemia". In addition, the processor may map, to blood glucose notification level 3, a normal state, a graphic 930, and a description "Blood glucose is stable. Maintain the current state". In addition, the processor may map, to blood glucose notification level 4, a hyperglycemic state, a graphic 940, and a description "Blood glucose is expected to be in the hyperglycemic range. Walking or climbing stairs can help lower blood glucose". In addition, the processor may map, to blood glucose notification level 5, a severe hyperglycemic state, a graphic 950, and a description "Blood glucose is expected to be in the hyperglycemic range. Check ketones.".

In addition, when the processor is unable to calculate the expected blood glucose value of the user, the processor may not assign a notification level or may map other level (e.g., unmeasurable), an insufficient-data state, a graphic 960, and a description "There is insufficient data for blood glucose prediction".

For example, cases where the processor is unable to calculate the expected blood glucose value may include a case where basic information of the user (e.g., the age and weight) is insufficient, a case where blood glucose data is not received from the blood glucose sensor, and a case where blood glucose data currently measured through the blood glucose sensor does not match blood glucose data calculated by the processor because the user did not input a meal bolus or the like.

FIG. 10 is an example for describing providing blood glucose notification cards according to the passage of a predetermined time period, according to an embodiment of the present disclosure.

Referring to FIG. 10, it may be assumed that blood glucose notification cards are provided every 5 minutes by using a graphic providing method linked to a blood glucose notification level. The processor may provide the user with a first blood glucose notification card 01 1010 about 20 minutes after starting to collect sensor glucose values (or blood glucose data) through the blood glucose sensor, and then provide the user with a blood glucose notification card 02 1020 and a blood glucose notification card 03 1030 at 5-minute intervals.

Assuming that blood glucose notification levels are classified based on an expected blood glucose value about 30 minutes from a reference time point (here, 30 minutes is an arbitrary time period), the processor may provide the user with a blood glucose notification card corresponding to a hyperglycemic state when the hyperglycemic state is expected in the near future even though the range of the blood glucose value obtained at the current time point corresponds to a normal state.

For example, referring to FIG. 10, the processor may obtain blood glucose values from about 4 sensors for about 20 minutes after the blood glucose sensors start obtaining sensor blood glucose values. Here, when a reference point 01 1001 is when 20 minutes elapse since the processor obtained first blood glucose data, the processor may provide the user with the first blood glucose notification card 01 1010 at a time point of about 50 minutes, which is 30 minutes (1011) after a reference time point 01.

That is, even when the blood glucose value at the current reference point 01 1001 corresponds to the normal range, the blood glucose notification card may classify the user after 30 minutes as a hyperglycemic state based on an expected blood glucose value after 30 minutes, and provide the corresponding blood glucose notification card 01 1010. Here, the applied threshold section of the blood glucose notification level is a section corresponding to about 200 mg/dl to 250 mg/dl, which corresponds to the hyperglycemic state.

Similarly, the processor may calculate, with a reference time point 02 1002 that is when about 25 minutes elapse, an expected blood glucose value at a time point of about 50 minutes, which is 30 minutes after (1021) the reference time point 02, based on immediately preceding sensor blood glucose values from the four sensors, classify a blood glucose notification level corresponding to the calculated blood glucose value as a high blood pressure state, and provide the user with the blood glucose notification card 02 1020 corresponding to the high blood pressure state.

Meanwhile, an insulin duration in the user's body may decrease at a certain rate according to the passage of a certain time period. Here, the processor may calculate, at a reference time point 03 1003 corresponding to a time point of about 30 minutes, an expected blood glucose value for the user at a time point of about 60 minutes, which is 30 minutes after (1031) the reference time point 03, and here, when the blood glucose value corresponds to a normal state, classify a normal state level, and provide the corresponding blood glucose notification card 03 1030.

FIG. 11 is another example of providing graphics linked to blood glucose notification levels, according to another embodiment of the present disclosure. Referring to FIG. 11, the graphics may be graphics representing a plurality of robots with different facial expressions, respectively. In addition, the processor may specify notification levels as three alerts: "Blood glucose range stable", "Blood glucose range concerned", and "Blood glucose range dangerous". Meanwhile, graphics to be output in other situations, such as 'unmeasurable', may also be specified.

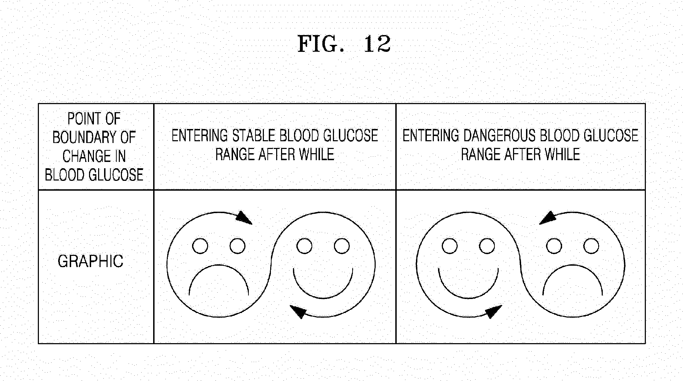

FIG. 12 is an example of providing graphics linked to boundaries of blood glucose notification levels, according to another embodiment of the present disclosure. For example, an expected blood glucose value may gradually change overtime. Accordingly, it may also be expected that a blood glucose notification level will change in a particular threshold section. The processor may store graphics that may be applied in a threshold section as illustrated in FIG. 12 to inform the user of a change in the blood glucose notification level. Referring to FIG. 12, the processor may further provide the user with a graphic for a case where the user's condition is expected to enter a stable range from a dangerous range after a while, or vice versa (a case where the user's condition is expected to enter a dangerous blood glucose range from the stable state after a while).

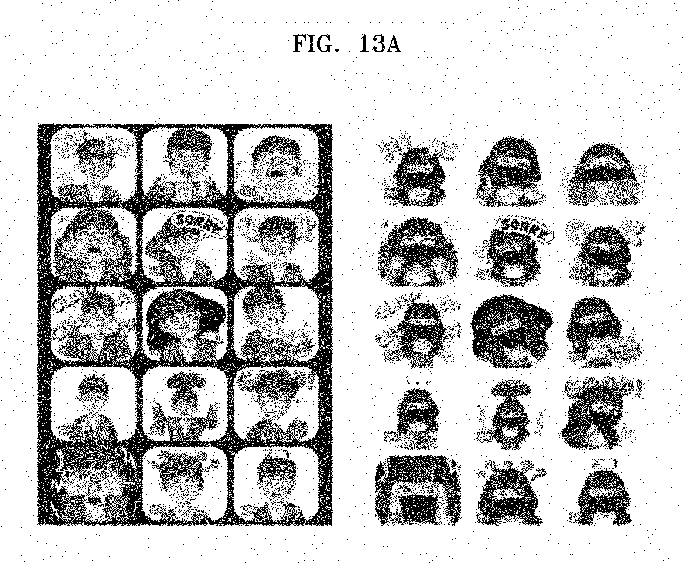

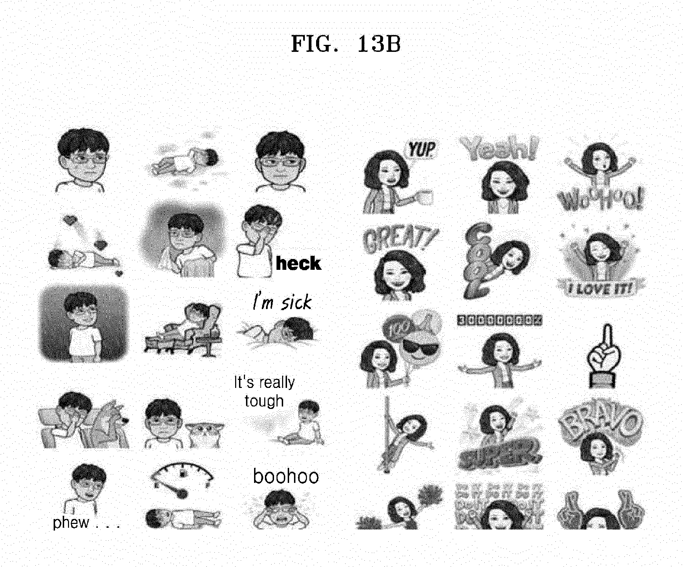

FIGS. 13A and 13B are examples of augmented reality (AR) emojis according to another embodiment of the present disclosure. According to an embodiment of the present disclosure, a graphic may be any one of the user's avatar, a virtual character, and an AR emoji generated by reflecting at least one of the user's appearance, shape, features, and preferences.

The user may specify in advance graphics linked to blood glucose notification levels. For example, the processor may set graphics representing an avatar that projects the user's appearance or shape, as graphics linked to blood glucose notification levels.

In addition, the processor may set, as a graphic, an AR emoji generated from a picture of the user by applying AR technology, a virtual character used in an online space, or the like. Here, the graphic is content created by an artist, and may be expressed in various manners. The present disclosure is not limited to the graphics illustrated in the drawings, and the scope of an embodiment including all essential components of the present disclosure may be determined by the scope of equivalent interpretation.

FIGS. 13A and 13B are examples of AR emojis according to different embodiments, respectively. Here, FIGS. 13B and 13B show different drawing styles and emoji expression formats, but may be explained together in that they are emojis created by reflecting at least one of the user's appearance, shape, features, and preferences.

Meanwhile, according to an embodiment of the present disclosure, a blood glucose notification level and a change in an emoji corresponding thereto do not need to have a sense of unity, and the emoji corresponding to the blood glucose notification level may be arbitrarily selected by the user.

An embodiment of the present disclosure may be implemented as a computer program that may be executed through various components on a computer, and such a computer program may be recorded in a computer-readable medium. In this case, the medium may include a magnetic medium, such as a hard disk, a floppy disk, or a magnetic tape, an optical recording medium, such as a compact disc read-only memory (CD-ROM) or a digital video disc (DVD), a magneto-optical medium, such as a floptical disk, and a hardware device specially configured to store and execute program instructions, such as ROM, random-access memory (RAM), or flash memory.

The processor may be configured to process a command of a computer program by performing basic arithmetic, logic, and input/output operations. The command may be provided to the processor by a storage medium or a communication module. For example, the processor may be configured to execute a command received according to program code stored in a recording device such as a storage medium.

Meanwhile, the computer program may be specially designed and configured for the present disclosure or may be well-known to and usable by those skilled in the art of computer software. Examples of the computer program may include not only machine code, such as code made by a compiler, but also high-level language code that is executable by a computer by using an interpreter or the like.

According to an embodiment, the method according to various embodiments of the present disclosure may be included in a computer program product and provided. The computer program product may be traded as commodities between sellers and buyers. The computer program product may be distributed in the form of a machine-readable storage medium (e.g., a CD-ROM), or may be distributed online (e.g., downloaded or uploaded) through an application store (e.g., Play StoreTM) or directly between two user devices. In a case of online distribution, at least a portion of the computer program product may be temporarily stored in a machine-readable storage medium such as a manufacturer's server, an application store's server, or a memory of a relay server.

The operations of the methods according to the present disclosure may be performed in any suitable order unless otherwise indicated herein or otherwise clearly contradicted by context. The present disclosure is not limited to the described order of the operations. The use of any and all examples, or exemplary language (e.g., 'and the like') provided herein, is intended merely to better illuminate the present disclosure and does not pose a limitation on the scope of the present disclosure unless otherwise claimed. Also, numerous modifications and adaptations will be readily apparent to those skilled in the art without departing from the spirit and scope of the present disclosure.

Accordingly, the spirit of the present disclosure should not be limited to the above-described embodiments, and all modifications and variations which may be derived from the meanings, scopes and equivalents of the claims should be construed as failing within the scope of the present disclosure.

### Industrial Applicability

An embodiment of the present disclosure may be used in the production of various drug injectors for injecting a drug into a human body.

## Claims

1. A method, performed by an alarm system linked to a drug injector, of differentially generating an alarm, the method comprising:
receiving state information from the drug injector;
determining priority of an alarm and a type of the alarm based on the received state information of the drug injector and a response scenario corresponding to the state information; and
differentially generating an alarm that stimulates at least one of senses of sight, hearing, and touch, based on the type and priority of the alarm, and the response scenario.

2. The method of claim 1, wherein the receiving of the state information comprises receiving state information regarding an operation or an error of the drug injector, through a communication module of the drug injector, or a communication module of a controller linked to the drug injector.

3. The method of claim 1, wherein a type of the response scenario comprises at least one of a response scenario related to a significant risk to a user's safety, a response scenario requiring an urgent process, a response scenario related to a predicted future situation, and a response scenario for managing the drug injector or the controller.

4. The method of claim 1, wherein the differentially generating of the alarm comprises:
first providing a visual alarm and a tactile alarm according to the determined priority and type of the alarm and then differentially providing an auditory alarm and a visual alarm, or
providing an auditory alarm along with the visual alarm and the tactile alarm.

5. The method of claim 1, wherein the differentially generating of the alarm comprises:
mapping an alarm expression method according to the determined priority and type of the alarm;
selectively combining alarm expression methods based on the response scenario; and
differentially providing the alarm to a user based on the combined alarm expression methods.

6. The method of claim 1, further comprising recommending an alarm providing method setting suitable for a user based on the user's alarm checking habits.

7. A computer-readable recording medium having recorded thereon a program for executing the method of claim 1 on a computer.

8. A method of providing a graphic linked to a blood glucose notification level, the method comprising:
obtaining current blood glucose data of a user through a blood glucose sensor;
calculating an expected blood glucose value over time based on the current blood glucose data of the user and an insulin duration for the user;
classifying a blood glucose notification level according to the expected blood glucose value at a particular time point at which a predetermined time period elapses from a current time point; and
outputting a graphic linked to the blood glucose notification level through a display screen.

9. The method of claim 8, wherein the blood glucose sensor is a continuous blood glucose meter to be attached to a human body to continuously measure blood glucose in the human body.

10. The method of claim 8, wherein the calculating of the blood glucose value comprises predicting an insulin duration for the user based on a plurality of pieces of blood glucose data obtained through the blood glucose sensor and user information that is input from the user, and calculate the blood glucose value by using a result of the predicting.

11. The method of claim 8, wherein the classifying of the blood glucose notification level further comprises presetting a threshold range of blood glucose values corresponding to a hypoglycemic state, a normal state, or a hyperglycemic state, and setting a blood glucose notification level that is mapped to a notification section, a notification method, and notification intensity for indicating a level of risk according to the state.

12. The method of claim 8, wherein the graphic is a visual indicator that changes in association with the blood glucose notification level as the expected blood glucose value changes over time.

13. The method of claim 8, wherein the graphic is an emoticon accompanied by a change in a facial expression, and
the outputting of the graphic linked to the blood glucose notification level comprises expressing a change in the facial expression of the emoticon in association with the blood glucose notification level.

14. The method of claim 8, wherein the graphic is an indicator having a shape of an arrow, and
the outputting of the graphic linked to the blood glucose notification level comprises determining a size, a color, and a shape of the arrow according to an increase or a decrease in the blood glucose value of the user, and expressing an animation effect of the arrow being added in association with the blood glucose notification level.

15. The method of claim 8, wherein the graphic is at least one of an avatar, a virtual character, and an augmented reality emoji of the user that are generated by reflecting at least one of an appearance, a shape, features, and preferences of the user, and
the outputting of the graphic linked to the blood glucose notification level comprises expressing a change in at least one of a facial expression, an action, and a background of the at least one of the avatar, the virtual character, and the augmented reality emoji of the user in association with the blood glucose notification level.
